# EUROPÄISCHE PATENTSCHRIFT

(11) **EP 0 842 648 B1**
(45) Veröffentlichungstag und Bekanntmachung des Hinweises auf die Patenterteilung: **30.07.2003**
(21) Anmeldenummer: 97117868.6
(22) Anmeldetag: 15.10.1997
(51) Int. Cl.: A61F 13/06

(54) **Selbstklebende Fertigbandage zur Band- und Muskelstabilisierung am Kniegelenk**
Adhesive bandage for ligament and muscle stabilization at the knee joint
Bandage adhésif pour ligament et muscle stabilisateurs de l'articulation du genou

(30) Priorität: 13.11.1996 DE 19646740
(43) Veröffentlichungstag der Anmeldung: 20.05.1998
(73) Patentinhaber: Beiersdorf Aktiengesellschaft, 20245 Hamburg (DE)
(72) Erfinder: Bodenschatz, Stefan, Dr., 21614 Buxtehude (DE); Himmelsbach, Peter, 21614 Buxtehude (DE)

(56) Entgegenhaltungen:
- EP-A- 0 741 998
- WO-A-88/01855
- DE-A- 3 843 483
- DE-A- 4 312 655
- US-A- 3 888 244
- US-A- 3 989 041

## Beschreibung

Die Erfindung betrifft eine einseitig selbstklebend beschichtete Fertigbandage zur Stützung und Stabilisierung des Kniegelenkaußenbandes (Lig. collaterale fibulare). Die dadurch bewirkte Entlastung des äußeren Seitenbandes dient insbesondere zur Behandlung von lateraler Instabilität durch atrophische Muskulatur, Kontusionen, Zerrungen, Überdehnungen oder leichten Einrissen der lateralen Bänder. Weiterhin kann die Bandage als Entlastungsverband eingesetzt werden für andere Indikationen, insbesondere für die Entlastung der ischiokruralen Muskulatur.

Die funktionelle Verbandtechnik, das sog. Taping, ist eine gängige Behandlungsmethode zur Prophylaxe und Therapie von Verletzungen, Erkrankungen und Veränderungen am Bewegungsapparat. Taping hat zum Ziel, die einzelnen Weichteile und Kapsel-, Band-Strukturen gezielt nachzubilden bzw. deren Funktionen selektiv zu stützen.

Der Tapeverband wird aus mehreren Bändern, sog. Zügeln, streifenweise unter Verwendung von vorzugsweise unelastischem Material, teilweise kombiniert mit elastischem Material, angelegt und erfüllt dann die Funktionen Stützen und Entlasten.

Derartige Verbände erfordern jedoch fachmännisches Können und sehr viel Erfahrung und können deshalb in aller Regel nicht vom Taping Unerfahrenen angelegt werden.

DE 4312655 A1 beschreibt eine Sprunggelenkbandage, die auf einer Seite selbstklebend beschichtet ist und zur Stützung des Srunggelenkes dient. Die Sprunggelenkbandage besitzt eine Längsrichtung und eine Querrichtung, bestehend aus einem im wesentlichen rechteckigen, in Querrichtung der Bandage mindestens teilweise unelastischen Teil A, an welchem in Längsrichtung gesehen nach oben und unten gerichtet jeweils zwei längliche Streifen B, C, D und E angeordnet sind, wobei der Teil A 2 bis 16 cm breit ist und die Streifen B, C, D und E 1 bis 8 cm breit sind.
Zum Unterschied umfasst die erfindungsgemäße Bandage, dass der mittlere Teil A wenigstens zu einem Teil in Querrichtung eine Höchstzugkraft-Dehnung von weniger als 30% aufweist und die Streifen B, C, D und E 30 bis 50 cm lang und 1 cm bis 8 cm breit sind.

Die Aufgabe die sich daraus ergibt ist eine Fertigbandage zur Verfügung zu stellen, die aufgrund ihrer Ausgestaltung, d.h. Design und Form, sowie der Materialeigenschaften zur Entlastung, Fixierung, Stabilisierung und/oder Funktionseinschränkung der Band- und Muskelstrukturen des Kniegelenkes, insbesondere des Außenbandes führt und vom Verwender auf einfache Weise, gegebenenfalls selbst, angelegt werden kann.

Gelöst wird diese Aufgabe durch eine Bandage gemäß Anspruch 1.

Der Mittelteil A ist, in Querrichtung der Bandage gesehen, vorzugsweise unelastisch ausgebildet, er kann jedoch auch nur teilweise unelastisch sein. Zum Beispiel in der Weise, daß das Material in dieser Richtung elastisch oder dehnbar ist, jedoch unelastische Verstärkungsstreifen aufweist. Der Teil A soll im angelegten Zustand, wie später noch gezeigt wird, im wesentlichen in Längsrichtung des Beines verlaufen. Die Querrichtung ist also die Wirkungsrichtung dieses Teiles der Bandage.

Der Mittelteil A besitzt als Ganzes bzw. zumindest teilweise in Querrichtung der Bandage eine Höchtzugkraft-Dehnung von weniger als 30%.

Die Streifen B, C, D und E können in einem Winkel ∝ von 30 bis 150°, bezogen auf die Querrichtung der Bandage, an dem Teil A angeordnet sein, wobei alle Variationen in der Ausrichtung der jeweiligen Streifen möglich sind. Vorzugsweise stehen sie jedoch senkrecht auf dem Mittelteil A, d.h. der Winkel ∝ beträgt 90° und die vier langen Streifen verlaufen parallel zueinander.

Die Länge der Streifen ist durch deren Anlegetechnik, die weiter unten noch genauer beschrieben wird, vorgegeben. Beispielhaft beträgt die Länge der Bandage insgesamt etwa 70-110 cm, vorzugsweise 100 cm, und sie ist etwa 2 - 16 cm, vorzugsweise 10 cm breit. Dabei ist das Mittelteil A ca. 10 cm lang und ca. 2- 16 cm, vorzugsweise 10 cm breit und die Streifen B, C, D, und E sind ca. 30 - 50 cm, vorzugsweise 45 cm lang und ca. 1 - 8 cm, vorzugsweise 5 cm breit Je nach Größenverhältnis des zu bandagierenden Gelenkes können sie auch länger und breiter sein oder gegebenenfalls gekürzt werden. Weiter können die Streifen (Zügel) unterschiedlich ausgebildet sein, d.h. beispielsweise können sie in der Dicke, Breite, Länge und/oder den textiltechnischen Kenndaten variieren oder aber auch partiell geteilt sein.

Die Bandage ist insgesamt einstückig ausgebildet, wobei sie entweder als Ganzes aus einem größeren Stück Bandagenmaterial ausgeschnitten, ausgestanzt oder aus Einzelteilen zusammengefügt wird.

Sie besteht besonders bevorzugt aus einem in einer Richtung dehnbaren oder elastischen textilen Material, vorzugsweise aus einem in einer Richtung dehnbaren oder elastischen Gewebe oder Gewirke insbesondere auf Baumwollbasis mit einer Höchstzugkraft von vorzugsweise mindestens 60 N/cm und einer Dehnfähigkeit bei einer Belastung von 10 N/cm bis zu etwa 90%, bevorzugt 10% bis 80%, in Längsrichtung. In der anderen Richtung beträgt die Höchstzugkraft-Dehnung maximal 30%. Das Material ist in der Bandage so ausgerichtet, daß das Mittelteil A in Querrichtung mindestens teilweise unelastisch ist.

Der Mittelteil A soll in Querrichtung mindestens teilweise unelastisch sein, so daß bei Herstellung der Bandage aus dem genannten Grundmaterial diese in Längsrichtung elastisch und in Querrichtung unelastisch ist

Als besonders günstig hat sich erwiesen, wenn sich am inneren Einschnitt zwischen den Streifen B/C und DIE eine kleine Ausnehmung befindet, um ein Einreißen zu verhindern und das Fixieren zu erleichtern.

Auf ihrer der Haut zugewandten Seite ist die Bandage mit einer der bekannten gut haftenden Selbstklebemassen beschichtet auf Basis von Kautschuk oder synthetischen Polymeren. Vorzugsweise sollte diese luft- und wasserdampfdurchlässig sowie gut hautverträglich sein.

Bis zum Gebrauch der Bandage kann die Klebeschicht mit einem klebstoffabweisend ausgerüsteten Blattmaterial, wie beispielsweise silikonisiertem Papier oder Kunststoff-Folie, abgedeckt sein.

Es hat sich dabei als günstig erwiesen, diese Abdeckung mehrteilig, vorzugsweise 5-teilig, durch z.B. perforierte Trennlinien, auszubilden. Dabei deckt ein Teil das Mittelteil A ab und 4 weitere streifenförmige Teile die schmaleren Streifen B, C, D und E. Die Abdeckungsteile können als Anbringungshilfe farbig markiert oder nummeriert sein.

Beim Anlegen der Bandage am gestreckten bzw. leicht gebeugten (<30°) Kniegelenk wird zuerst der Mittelteil A auf der Außenseite des Kniegelenks fixiert. Dieses geschieht so, daß er der Breite nach - also in Querrichtung - distal vom Oberschenkelansatz zum Unterschenkelansatz verklebt wird. Die Streifen B, C, D, E, die in der Fachsprache Zügel genannt werden, bilden einen 90° Winkel zum Bein. Dann wird der Streifen B rückseitig von lateral zirkulär nach medial am Oberschenkel geführt und angeklebt, so daß er zumindest einen Teil, bevorzugt den ganzen Oberschenkel wenigstens einmal umschließt. Als nächster Zügel wird der Streifen C angelegt. Dieser verläuft ebenfalls rückseitig von lateral zirkulär nach medial am Unterschenkel. Ebenfalls soll dieser Zügel einen Teil, bevorzugt den gesamten Unterschenkel wenigstens einmal umschließen. Bei beiden Zügeln B und C ist darauf zu achten, daß der Kniekehlenbereich nicht verklebt wird.

Der Zügel D wird so angebracht, daß er frontseitig distal zum Unterschenkel verläuft und dort von medial zirkulär nach lateral verklebt wird. Der Zügel soll zumindest einen Teil, bevorzugt den ganzen Unterschenkel wenigstens einmal umschließen.

Zuletzt wird der Zügel E angelegt Er wird proximal vom Unterschenkel zum Oberschenkel und dort von medial kommend zirkulär nach lateral geführt

Die klebstoffabweisenden Abdeckmaterialien auf den Streifen werden entsprechend dem Vorgehen beim Ankleben der Reihe nach entfernt. Die Bandage kann zusätzlich durch Ankerstreifen aus üblichem Tapematerial verstärkt werden.

Die fertig angelegte Bandage stützt, fixiert, stabilisiert und entlastet die Bandund Muskelstrukturen des Kniegelenkes, insbesondere des Außenbandes, wobei die Stabilisierung des Gelenkes durch den in Querrichtung unelastischen Teil des Streifens A gewährleistet wird, die Streifen B, C, D, E fixierend und unterstützend wirken.

Ein vereinfachtes Anlegen der Fertigbandage ist jedoch auch möglich. Hierbei werden die Zügel D und E nur zirkulär um den Unter- bzw. Oberschenkel geführt. Durch ein Überlappen der Zügel BID und CIE wird die Fixierung des Mittelteils A auch bei starker Belastung gewährleistet. Besonders einfach läßt sich dieses Überlappen durch eine Dreiecksform der Abdeckung auf der selbstklebenden Seite des Mittelteils A erreichen. Hierbei können die Zügel B und C noch unter dem Mittelteil A geführt werden. Dieses verstärkt die Fixierung.

Wird der Verband für andere Muskel- oder Bandstrukturen verwendet, so ist die Bandage so anzulegen, daß die Orientierung der Querrichtung des Mittelteils A entsprechend der zu unterstützenden Struktur verläuft. Zum Beispiel ist die Kniekehle der Startpunkt für einen Entlastungsverband für die ischiokrurale Muskulatur. Das Anlegen der Zügel B, C, D und E der Fertigbandage ist analog dem oben beschriebenen vereinfachten Anlegen durchzuführen, indem diese zirkulär um den Unter- bzw. Oberschenkel geführt werden.

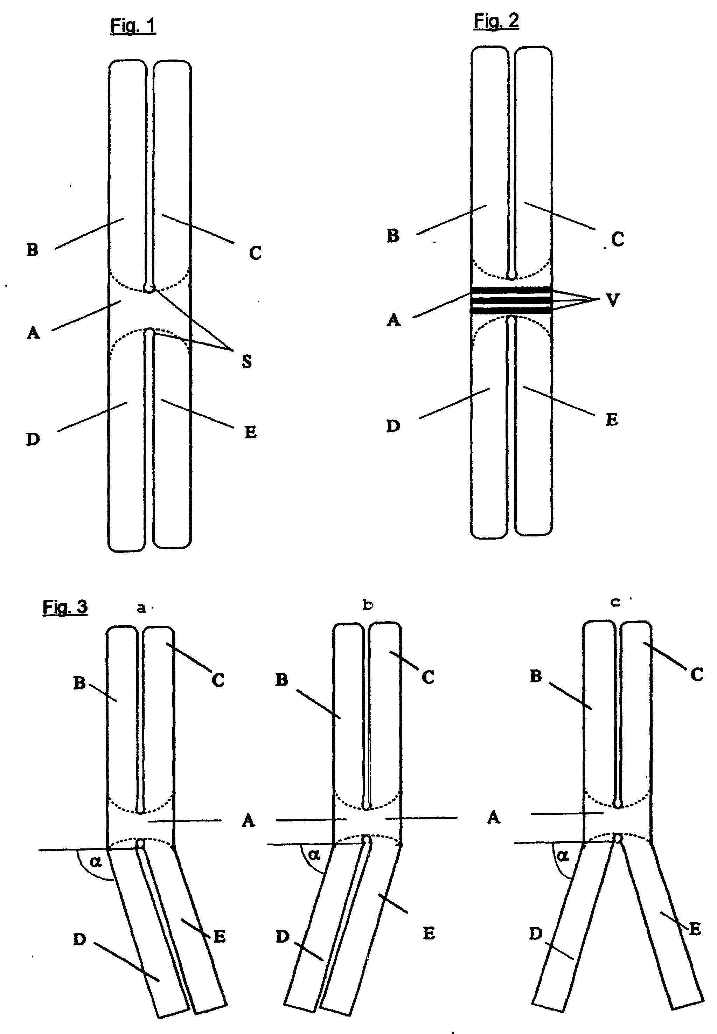

Die erfindungsgemäße Bandage ist in Figur 1 beispielsweise dargestellt, wobei die länglichen Streifen (Zügel) B, C, D und E senkrecht zu dem Mittelteil A angeordnet sind. D.h., der Winkel ∝ beträgt 90°. Mit S ist der Schnittpunkt von A, B und C bzw. A, D und E bezeichnet. Die gestrichelten Linien bedeuten die Perforation in der Abdeckung auf der klebenden Seite der Bandage.

Figur 2 zeigt beispielhaft eine längs- und querelastische Bandage mit drei unelastischen Verstärkungsstreifen (V), die im Mittelteil A in Querrichtung mit der Bandage verbunden sind. Der Mittelteil A ist dadurch in Querrichtung teilweise unelastisch und damit erfindungsgemäß wirksam.

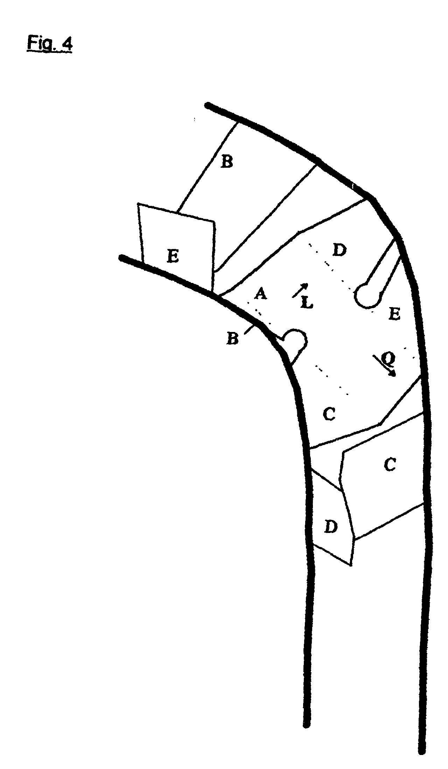

Figur 3 zeigt unter a, b und c drei Variationen der Anordnung der Zügel B, C, D und E mit einem Winkel ∝ der Zügel D und E zur Querrichtung der Bandage von jeweils ca. 115° bei a und jeweils ca. 65° bei b sowie ca. 65° für D und ca. 115° für E bei c.

Figur 4 zeigt die Bandage im angelegten Zustand mit einer Wickelung wie weiter oben beschrieben.

## Patentansprüche

1. Auf einer Seite selbstklebend beschichtete Bandage zur Entlastung und Stabilisierung von Band- und Muskelstrukturen am Kniegelenk, die eine Längsrichtung und eine Querrichtung hat, bestehend aus einem rechteckigen, in Querrichtung der Bandage mindestens teilweise unelastischen Teil A, an welchem in Längsrichtung gesehen nach oben und unten gerichtet jeweils zwei längliche Streifen B, C, D und E angeordnet sind, wobei der Teil A 2 bis 16 cm breit ist und die Streifen B, C, D und E 1 bis 8 cm breit sind,
**dadurch gekennzeichnet, dass** der Streifen A 10 cm lang und wenigstens zu einem Teil in Querrichtung eine Höchstzugkraft-Dehnung von weniger als 30% aufweist und die Streifen B, C, D und E 30 bis 50 cm lang sind.

2. Bandage nach Anspruch 1, **dadurch gekennzeichnet, dass** die Streifen B, C, D und E in einem Winkel ∝ von 30 bis 150° bezogen auf die Querrichtung der Bandage an dem Teil A angeordnet sind.

3. Bandage nach Anspruch 1 oder 2, **dadurch gekennzeichnet, dass** der Winkel ∝ 90° beträgt.

4. Bandage nach einem der vorhergehenden Ansprüche, **dadurch gekennzeichnet, dass** der Streifen A 10 cm breit ist und die Streifen B, C, D und E 45 cm lang und 5 cm breit sind.

5. Bandage nach einem der vorhergehenden Ansprüche, **dadurch gekennzeichnet, dass** sie aus einem in einer Richtung dehnbaren oder elastischen textilen Material besteht.

6. Bandage nach einem der vorhergehenden Ansprüche, **dadurch gekennzeichnet, dass** die Streifen unterschiedlich dick und/oder unterschiedlich elastisch sind.

7. Bandage nach einem der vorhergehenden Ansprüche, **dadurch gekennzeichnet, dass** sich am inneren Schnittpunkt S von A, B und C bzw. A, D und E zwischen diesen Teilen eine Ausnehmung befindet.

8. Bandage nach einem der vorhergehenden Ansprüche, **dadurch gekennzeichnet, dass** sie auf ihrer klebstoffbeschichteten Seite mit einem klebstoffabweisenden Material abgedeckt ist.

9. Bandage nach Anspruch 8, **dadurch gekennzeichnet, dass** das klebstoffabweisende Trägermaterial 5-teilig ausgebildet ist, wobei je ein Teil die Streifen A, B , C, D und E abdeckt.

## Claims

1. Bandage self-adhesively coated on one side, for relieving and stabilizing ligaments and muscle structures at the knee joint, which has a longitudinal direction and a transverse direction, comprising a rectangular part A which is at least partially inelastic in the transverse direction of the bandage and on which there are respectively arranged, directed upwards and downwards seen in the longitudinal direction, two elongate strips B, C, D and E, the part A being 2 to 16 cm wide and the strips B, C, D and E being 1 to 8 cm wide, **characterized in that** the strip A is 10 cm long and has at least partially a maximum elongation under tensile force of less than 30% in the transverse direction and the strips B, C, D and E are 30 to 50 cm long.

2. Bandage according to Claim 1, **characterized in that** the strips B, C, D and E are arranged on the part A at an angle ∝ [sic] of from 30 to 150° with respect to the transverse direction of the bandage.

3. Bandage according to Claim 1 or 2, **characterized in that** the angle ∝ [sic] is 90°.

4. Bandage according to one of the preceding claims, **characterized in that** the strip A is 10 cm wide and the strips B, C, D and E are 45 cm long and 5 cm wide.

5. Bandage according to one of the preceding claims, **characterized in that** it consists of a textile material which is extensible or elastical in one direction.

6. Bandage according to one of the preceding claims, **characterized in that** the strips are of different thicknesses and/or of different elasticities.

7. Bandage according to one of the preceding claims, **characterized in that** a clearance is located at the inner point of intersection S of A, B and C or A, D and E between these parts.

8. Bandage according to one of the preceding claims, **characterized in that** it is covered on its adhesive-coated side with an adhesive-repellent material.

9. Bandage according to Claim 8, **characterized in that** the adhesive-repellent substrate material is designed in 5 parts, one part in each case covering the strips A, B, C, D and E.

## Revendications

1. Bandage autocollant sur une face, pour le soulagement et la stabilisation de structures de ligament et de muscles de l'articulation du genou, qui possède une direction longitudinale et une direction transversale, constitué d'une partie A rectangulaire, au moins en partie non élastique dans la direction transversale du bandage et sur laquelle, vu dans le sens de la longueur, deux bandes allongées respectives B, C, D et E sont disposées vers le haut et vers le bas, la partie A présentant une largeur de 2 à 16 cm et les bandes B, C, D et E présentant une largeur de 1 à 8 cm,
**caractérisé en ce que** la bande A a une longueur de 10 cm et présente au moins en partie dans la direction transversale un allongement sous la force maximale de traction inférieur à 30%, les bandes B, C, D et E ayant une longueur de 30 à 50 cm.

2. Bandage selon la revendication 1, **caractérisé en ce que** les bandes B, C, D et E sont disposées sur la partie A sous un angle α de 30 à 150° par rapport à la direction transversale du bandage.

3. Bandage selon la revendication 1 ou 2, **caractérisé en ce que** l'angle α vaut 90°.

4. Bandage selon l'une des revendications précédentes, **caractérisé en ce que** la bande A présente une largeur de 10 cm, les bandes B, C, D et E ayant une longueur de 45 cm et une largeur de 5 cm.

5. Bandage selon l'une des revendications précédentes, **caractérisé en ce qu'**il est constitué d'un matériau textile, extensible ou élastique dans une direction.

6. Bandage selon l'une des revendications précédentes, **caractérisé en ce que** les bandes ont une épaisseur différente et/ou une élasticité différente.

7. Bandage selon l'une des revendications précédentes, **caractérisé en ce qu'**au point intérieur de concours S entre A, B et C et entre A, D et E, une découpe est prévue entre ces parties.

8. Bandage selon l'une des revendications précédentes, **caractérisé en ce qu'**il est recouvert d'un matériau non adhésif sur son côté recouvert d'adhésif.

9. Bandage selon la revendication 8, **caractérisé en ce que** le matériau de support non adhésif est configuré en 5 pièces, une pièce respective recouvrant chacune des bandes A, B, C, D et E.
